# EUROPEAN PATENT APPLICATION

(11) **EP 4 553 717 A1**
(43) Date of publication of application: **14.05.2025**
(21) Application number: 23208851.8
(22) Date of filing: 09.11.2023
(51) Int. Cl.: G06N 5/04, G16H 50/20, G16H 30/00, G06F 16/55

(54) **METHOD FOR PERFORMING A CHECK OF A STACKED SYSTEM**

(71) Applicant: Siemens Healthineers AG, 91301 Forchheim (DE)
(72) Inventor: Biniazan, Ramyar, 90402 Nürnberg (DE); Fieselmann, Andreas, 91052 Erlangen (DE); Hümmer, Christian, 96215 Lichtenfels (DE)
(74) Representative: Siemens Healthineers Patent Attorneys

(57) **Abstract**

The invention relates to a computer implemented method for performing a check of a stacked system (STACK-SYS). The method comprises a step of receiving (REC)input data (IO). The method further comprises a step of applying (APP-1) a first function (M1, M1.1, M1.2, M1.3) to the input data, wherein an intermediate output data (IO1, IO2, IO3) is generated. The method further comprises a step of performing (CHECK) a check based on the intermediate output data (IO1, IO2, IO3). If the check is positive, the method comprises the steps of providing (PROV-1) the intermediate output data (IO1, IO2, IO3) to a second function (M2) at least as part of an intermediate input data, applying (APP-2) the second function (M2) to the intermediate input data, wherein an output data is generated, and providing (PROV-2) the output data.

## Description

The invention relates to a method for performing a check of a stacked system, a stacked system, a system for performing a check of a stacked system, a computer program product, and a computer-readable storage medium.

Stacked systems are well known in the art. A stacked system consists of at least a first and a second function. Therein typically at least one of the functions comprises a trained function. The first function is applied to an input data, generating an intermediate output data. This intermediate output data is used as input data for the second function, which generates a further intermediate output data for a third function or the final output data.

Like this, the first system can be a more general function which can be used to prepare the input data for a more specific task. The second function can be an application specific function which is configured for a specific task in a specific environment.

In particular, with regard to trained functions this allows to use the more general first function which can be applied in several use cases. Furthermore, the first function can be trained with a huge amount of less specific data. The second function can then be trained on a smaller amount of data specific for the use case respectively the application. For example, the first function can be configured to segment organs in a medical image data. The second function can then be specific for answering a specific clinical question concerning one of the segmented organs.

Nevertheless, if the intermediate output is incorrect, this leads to an incorrect final output of the second function. For a user it is often not obvious where and when the mistake occurred. In particular in the medical context, this causes problems, as the reliability of systems used in the medical context is an especially crucial factor.

It is the object of the present invention to provide a method for enhancing the reliability of such a stacked system.

This object is achieved by a method for performing a check of a stacked system, a stacked system, a system for performing a check of a stacked system, a computer program product, and a computer-readable storage medium according to the independent claims. Advantageous features and further developments are listed in the dependent claims and in the following specification.

In the following the solution according to the invention is described with respect to the claimed systems as well as with respect to the claimed methods. Features, advantages, or alternative embodiments herein can be assigned to the other claimed objects and vice versa. In other words, claims for the systems can be improved with features described or claimed in the context of the methods. In this case, the functional features of the method are embodied by objective units of the system.

Furthermore, in the following the solution according to the invention is described with respect to methods and systems for performing a check of a stacked system as well as with respect to methods and systems for providing a trained function. Features, advantages, or alternative embodiments herein can be assigned to the other claimed objects and vice versa. In other words, claims for methods and systems for providing the trained function can be improved with features described or claimed in context of the methods and systems for performing a check of a stacked system, and vice versa.

In particular, the trained function used within the methods and systems for performing a check of a stacked system can be adapted by the methods and systems for providing the trained function. Furthermore, input data used in the methods and systems for performing a check of a stacked system can comprise advantageous features and embodiments of training input data used in the methods and systems for providing the trained function, and vice versa. Furthermore, output data used in the methods and systems performing a check of a stacked system can comprise advantageous features and embodiments of output training data used in the methods and systems for providing the trained function, and vice versa.

Independent of the grammatical term usage, individuals with male, female or other gender identities are included within the term.

In a first aspect the invention relates to a computer implemented method for performing a check of a stacked system. The method comprises a step of receiving input data. The method furthermore comprises a step of applying a first function to the input data, wherein an intermediate output is generated. The method comprises a further step of performing a check based on the intermediate output data. If the check is positive, the method comprises a step of providing the intermediate output data to a second function at least as part of an intermediate input data, applying the second function to the intermediate output data, wherein an output data is generated and providing the output data.

The input data comprises in particular a medical image data. The medical image data depicts at least a part of a patient. The patient can be a human or an animal or an object. The medical image data comprises in particular a number of pixels, wherein each pixel comprises a pixel value. Therein, the first function can be applied on each pixel value.

The input data is received by an interface. The input data can be provided by a database and/or by a storage medium and/or by a medical imaging apparatus like an x-ray system or a magnet resonance system or a computed tomography system or an ultrasound system.

By applying the first function to the input data an intermediate output data is generated. The intermediate output data can also comprise an image data. Alternatively, the intermediate output data can comprise any other data, like a vector data or a single value or coordinates which relate to the pixels of the inputted medical image data etc.

Based on this intermediate output data a check is performed. During the check it is checked respectively approved whether the intermediate output data is consistent with a rule or prior knowledge. In particular, during the check the intermediate output data is compared with a rule and/or prior knowledge about expected output data. The check can be performed automatically. Alternatively or additionally, the check can be performed manually based on a user input.

In particular, the check can comprise applying a trained function to the intermediate output data. The output of this trained function indicates whether the check is positive or negative.

A positive result of the check means, that the intermediate output data fulfills the rules and/or is consistent with the prior knowledge and, therefore, seems to be correct. A negative result of the check means that the intermediate output data at least partly does not fulfill the rules or is at least partly not consistent with the prior knowledge.

If the check is positive, the intermediate output data is provided to the second function at least as part of intermediate input data. In particular, the intermediate input data can comprise further data in addition to the intermediate output data as for example the input data. In particular the intermediate input data can comprise intermediate output data of a third function. The second function is applied to the intermediate input data. Thereby, the output data is generated. The output data is provided. In particular the output data can be provided as final output data to a user via a user interface and/or to a database. Alternatively or additionally, the output data can be provided as second intermediate output data to a third function which is applied to the output data, thereby generating another output data.

The invention is not limited to just one first and one second function. The stacked system can comprise more than one first function and/or more than one second function. In particular, the stacked system can comprise further functions which follow the first and second function(s) and which are applied to the output data of at least one of the first and/or second function(s) if the checks are positive.

The method has the advantage that each intermediate output data is checked whether it is correct or not, in the sense that it is checked whether it is consistent with rules and/or prior knowledge and/or user knowledge. Based on that it can be avoided that faulty intermediate output data is further processed by a further function of the stacked system which might lead to severely false final output data. At least the user is informed about any problem which is detected during the check by a warning message. Hence, this helps to improve the explainability of a stacked system. Therefore, the reliability of such stacked systems can be improved which is in particular in medical environment a crucial factor.

According to an aspect of the invention, if the check is negative, the method comprises a step of providing a warning message.

If the check is negative, meaning that the intermediate output data seems to be faulty, a warning message is provided. This warning message can in particular be provided to a user via a user interface. The user interface can in particular be a monitor and/or a speaker and/or a light. The warning message can be configured to stop further processing the intermediate output data. Alternatively, the warning message can be just an information to the user, that there occurred a problem during the check.

Even if the check is negative and a warning message is provided optionally, the intermediate output data can be provided to the second function as described above for the case of a positive check and the second function can be applied to the intermediate output data. The thereby generated output data can then be provided as described above.

Advantageously, the user is informed about a problem during the check. Even if the intermediate output is used as input for the second function, the user knowns, that he has to check the final output more carefully, as it is not safe, that everything worked correctly.

According to an aspect of the invention the first function comprises a first trained function and/or the second function comprises a second trained function.

In general, the trained function mimics cognitive functions that humans associate with other human minds. In particular, by training based on training data the trained function is able to adapt to new circumstances and to detect and extrapolate patterns.

In general, parameters of the trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, the trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network, or a convolutional deep neural network. Furthermore, a neural network can be an adversarial network, a deep adversarial network and/or a generative adversarial network.

Each of the trained functions, in particular the first and the second trained function, are trained independently from each other.

That means, that a first training input data and a first training output data are used to train the first function. For training the first function is applied to the first training input data, wherein a first estimated output data is generated. This first estimated output data is compared with the first training output data and at least one parameter of the first trained function is adapted such that, when the first trained function is again applied on the first training input data, the first estimated output data, which is determined based on this adapted first trained function is more similar to the first training output data. The first training input data has the same characteristics as the input data.

The second trained function is trained based on a second training input data and a second training output data. The second training input data can comprise the first training output data. The second trained function is trained analogously to the first trained function based on the corresponding training data.

The inventors recognized that a stacked system is in particular beneficial if trained functions are used. Like this, it is possible, to train distinct functions for different use cases based on different and more or less specific training data. Like this for example, the first trained function can be trained more general than the second trained function. That means, that the second trained function can be more application specific whereas the first trained function is more a preprocessing step of the input data of the second trained function.

According to a further aspect of the invention providing the output data comprises at least one of the following steps: providing the output data as final output data to a user and/or a database and/or an imaging device, or providing the output data as second intermediate input data to a third function after performing a check.

If the output data is the final output data of the stacked system, it can be provided to a user via a user interface and/or to a database and/or to an imaging device.

The user interface can in particular be a monitor which can display the final output data. The user can in particular be a medically skilled person like a medical doctor and/or a medical assistance. Alternatively, the user can be a patient.

The database can in particular be a radiology information system (acronym: RIS) and/or a picture archiving and communication system (acronym: PACS) and/or a hospital information system (acronym: HIS) and/or a laboratory information system (acronym: LIS) etc. The final output data can in particular be integrated in an electronic medical patient record which is saved in one of the above-mentioned databases and which corresponds to the patient, the input data belongs to. In this case, the final output data can in particular comprise a diagnosis and/or a treatment proposal etc.

The imaging device can in particular be a medical imaging device like an x-ray system and/or a magnetic resonance system and/or a computed tomography system and/or an ultrasound system. In this case, the final output data can in particular comprise at least one imaging parameter which is configured to be used to control the imaging device while acquiring a new image. The imaging parameter can be configured to control an image acquisition with the medical imaging device.

If the output data is just a second intermediate output data a further check, which can be configured as described above, is performed based on this second intermediate output data. If this check is positive, the second intermediate output data can be provided at least as part of a second intermediate input data to a third function. The third function can comprise a third trained function. The second intermediate input data can also comprise the input data of the stacked system.

If the check is negative, optionally a warning message is provided. As described above, also in this case, optionally, the second intermediate output data can be provided to the third function.

Advantageously the output data is configured such that it can be further used. For that purpose, the output data is provided in the corresponding manner.

According to a further aspect of the invention the method further comprises at least one of the following steps if the output data is provided as final output data: acquiring medical image data with a medical imaging system based on the output data, automatically including the output data in a patient record, providing a diagnosis based on the output data.

If a medical image data is acquired based on the final output data, the final output data defines at least one acquisition parameter. The medical imaging system can for example comprise at least one of the following an x-ray system, a computed tomography system, a magnetic resonance system, an ultrasound system. The final output data is then provided to the medical imaging system such that the medical imaging system can acquire a medical image data based on the final output data.

Alternatively or additionally, the final output data can automatically be included in a patient record. The patient record is in particular an electronic patient record. The patient record relates to a patient. The input data is related to the same patient as the patient record.

Alternatively or additionally, a diagnosis can be provided based on the final output data. This diagnosis can be included in the patient record as above described. Alternatively or additionally, the diagnosis can be provided to a user via a user interface in particular via a monitor. The diagnosis can comprise an information about a disease the patient to whom the input data relate to, most probably has. Alternatively or additionally, the diagnosis needs not necessarily relate to an illness or an anomaly. In particular, the diagnosis can also describe physical facts of the patient, e.g., the diagnosis can describe a status of the patient for example the form of the spine etc.

The inventors recognized that the final output data can be further used.

According to a further aspect of the invention the check is rule-based.

Therein, at least one rule is defined an advance, which the intermediate output data has to fulfill. This rule can be based on prior knowledge about the expected output data. Alternatively, the rule can be learned based on a number of intermediate output data. With other words, the rule can be an expectation value based on a huge sample of values for the intermediate output data. The rule can define within which range around the expectation value, the intermediate output data fulfills the rule and when it is out of the range and thus, the rule is not fulfilled.

If the first function for example comprises a segmentation of organs and/or bones in a medical image a rule can for example specify that the number of segmented kidneys is two. Another rule can for example specify the expected range of distances between different vertebrae of the spine. A vast number of such rules is possible which define expected ranges for the intermediate output data.

The inventors recognized that based on the rule-based check it can be proved whether the intermediate output data fulfills expectations/rules which can be based on prior knowledge and define typical ranges for the intermediate output data. Like this, it is possible to detect deviations of the intermediate output data.

According to a further aspect of the invention performing the check comprises a step of providing the intermediate output data to a user and a step of receiving a user input based on the provided intermediate output data. Therein the user input indicated whether the check is positive or negative.

The intermediate output can be provided to the user via a user interface. The user interface can at least comprise a monitor. The intermediate output data can then be displayed via the monitor.

The user input can also be received via the user interface. For this purpose, the user interface can comprise at least one of the following: a keyboard, a computer mouse, a touchscreen, a touch pad, a microphone for voice control etc. The user input can indicate whether the intermediate output data is correct or not. If the intermediate output data is correct, the check is positive. If the intermediate output data is incorrect, the check is negative.

The inventors recognized that it can be beneficial to include a manual component in the check. Like in particular if there are deviations of the intermediate output data from the standard/the prior knowledge, it can be checked, whether this deviation is correct or not. For example, some humans only have one kidney. In the above-mentioned example, when the first function comprises a segmentation algorithm, it can be checked by the user, whether it is correct that only one kidney is segmented, because the patient only has one kidney or whether it is incorrect that only one kidney is segmented and the first function failed to segment the second kidney. The inventors recognized that including the user in the check helps to determine a faulty intermediate output data to avoid that the final output data is incorrect as a consequence.

According to a further aspect of the invention, the intermediate output data is provided to the user if the rule-based check is negative.

With other words, the check comprises two steps. In a first step the above-described rule-based check is performed based on the intermediate output data. If this rule-based check is positive, then the intermediate output data is provided to the second function at least as part of the intermediate input data. If this rule-based check is negative, a second step of the check follows. This second step comprises a manual, user-based check, as described above. In this case, the user can check, via the user interface, whether the rule-based check was correct or whether the rule-based check failed e.g., because the intermediate output data comprises an exception to the rule. If the user finds that the rule-based check has been correct, then the check is negative. If the user finds that the rule-based check failed, then the check is positive.

Exemplarily the input data comprises a medical image data depicting a patient with one kidney. The first function comprises an organ segmentation function. This first function segments only one kidney. The rule-based check is negative because the corresponding rule says that humans have two kidneys. Thus, the segmented medical image data is provided to the user. The user can check whether there is one further kidney, which is not segmented or whether the depicted patient really has only one kidney. If the patient has only one kidney, the user can indicate via the user input, that the first function was correct. In this case, the check is positive.

The inventors recognized that only selected cases should be provided to the user in order to avoid a work overload of the user. Advantageously, only those cases are provided to the user, where possibly mistakes occur. The user is in this case a double-checking instance for unclear cases. Like this it can be avoided that correct deviations from the standard are classified as false and the stacked system is stopped, on the other hand, it can be avoided that the user has to check each and every intermediate output data.

According to a further aspect of the invention, the user input comprises one of the following: an agreement of the user with the intermediate output data, indicating a positive check, a disagreement of the user with the intermediate output data, indicating a negative check, a correction of the intermediate output data, wherein the corrected intermediate output data replaces the intermediate output data during the following procedure, indicating a positive check based on the corrected intermediate output data.

If the user agrees with the intermediate output data, the user indicates, that the intermediate output data is correct. In this case, the check is positive.

If the user disagrees with the intermediate output data, the user indicates, that the intermediate output data is faulty. In this case, the check is negative.

As a third option the user can correct the intermediate output data. For example, the user can manually correct a faulty segmentation. This corrected intermediate output data replaces the intermediate output data in the following proceeding. The check is then assumed to be positive. The corrected intermediate output data is then provided to the second function at least as part of the intermediate input data.

The inventors recognized that the user input can be used as check. The inventors furthermore recognized that it can be helpful if the user can correct the intermediate output data in order to be able to further execute the stacked system with the corrected data. Like this, follow-up mistakes of the final output data can be avoided while the advantages of the stacked system can be used.

According to a further aspect of the invention the input data comprises a medical image data, in particular a medical radiology image data.

A medical image data in particular depicts at least a part of a patient. The patient can be a human or an animal. In particular, the medical image data depicts an inner structure of the patient.

A medical radiology image data can in particular comprise an x-ray image and/or a computed tomography image and/or a magnetic resonance image.

The inventors recognized that it is beneficial to apply the stacked system to medical image data. With other words, a stacked system can be used to process medical image data. The inventors recognized that the check can be performed onto intermediate output which results by applying the first function on medical image data.

According to a further aspect of the invention the input data comprises a medical image data of a spine. Therein the intermediate output data comprises at least one of the following: a position of at least one vertebrae of the spine, a segmentation of the spine, a baseline describing the form of the spine.

With other words, the stacked system is configured to analyze the spine of the patient. The first function can then be configured to determine the position of at least one vertebrae of the spine. Alternatively or additionally, the first function can be configured to segment the spine, in particular the vertebrae of the spine. Alternatively or additionally, the first function is configured to determine a baseline of the spine, describing the form of the spine.

The inventors recognized that the described method can be advantageously used to analyze a spine of a patient.

In a second aspect the invention relates to a stacked system comprising at least one first function, at least one checking gate and at least one second function. Therein the at least one first function is configured for being applied to input data, therein generating intermediate output data. Therein the at least one checking gate is configured for performing a check of the intermediate output data. And if the check is positive, the second function is configured for being applied to the intermediate output data thereby generating an output data.

The stacked system is in particular configured to be used in the above-described method.

According to the aspect of the invention, the check is rule-based and/or based on a user input.

In particular, the check is configured as described above.

In a third aspect, the invention relates to a system for performing a check of a stacked system comprising an interface and a computation unit. Therein the interface and the computation unit are configured to perform the following steps:
- receiving input data,
- applying a first function to the input data,
   wherein an intermediate output data is generated,
- performing a check based on the intermediate output data,
   if the check is positive,
- providing the intermediate output data to a second function at least as part of an intermediate input data,
- applying the second function to the intermediate input data, wherein an output data is generated,
- providing the output data, or
   if the check is negative,
- providing a warning message.

In particular, the system can be configured to execute the previously described method for performing a check of a stacked system. The system is configured to execute this method and its aspects by the interface and the computation unit being configured to execute the corresponding method steps. In particular, the interface can comprise one or more sub-interfaces. In particular, the computation unit can comprise one or more computation sub-units.

In a further aspect the invention relates to a training system for providing a stacked system, wherein the stacked system comprises a first and a second function. Wherein the first function comprises a first trained function and/or the second function comprises a second trained function. The training system comprises a training interface and a training computation unit which are configured to perform the following steps:
if the first function comprises a first trained function:
   - receiving first input training data and first output training data,
   - training the first function based on the first input training data and the first output training data,
if the second function comprises a second trained function:
   - receiving second input training data and second output training data,
wherein the first output training data is identical to the second input training data,
   - training the second function based on the second input training data and the second output training data,
   - providing the stacked system comprising the first and the second function.

In a fourth aspect the invention relates to a computer program product with a computer program and a computer-readable medium. A mainly software-based implementation has the advantage that even previously used systems can be easily upgraded by a software update in order to work in the manner described. In addition to the computer program, such a computer program product can optionally include additional components such as documentation and/or additional components, as well as hardware components such as e.g., hardware keys (dongles etc.) for using the software.

In a further aspect the invention relates to a computer program product comprising program elements directly loadable into a memory unit of a first providing system, which induces the system to execute the method according to the claimed method and its aspects when the program elements are executed by the system.

In a fifth aspect the invention relates to a computer-readable storage medium comprising program elements which are readable and executable by a system, to execute the claimed method and its aspects, when the program elements are executed by the system.

In a further aspect the invention relates to a computer-readable storage medium, comprising a stacked system as claimed.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Figure 1 displays a schematic flowchart of an exemplary stacked system as known in the prior art,
Figure 2 displays a schematic flow chart of a first embodiment of the method for performing a check of a stacked system,
Figure 3 displays a schematic flow chart of a first embodiment of a step of performing a check,
Figure 4 displays a schematic flow chart of a second embodiment of a step of performing a check,
Figure 5 displays a schematic flow chart of a third embodiment of a step of performing a check,
Figure 6 displays a schematic flowchart of a stacked system comprising a checking gate,
Figure 7 displays a schematic flowchart of processing data by applying the method for performing a check of a stacked system,
Figure 8 displays a system for performing a check of a stacked system.

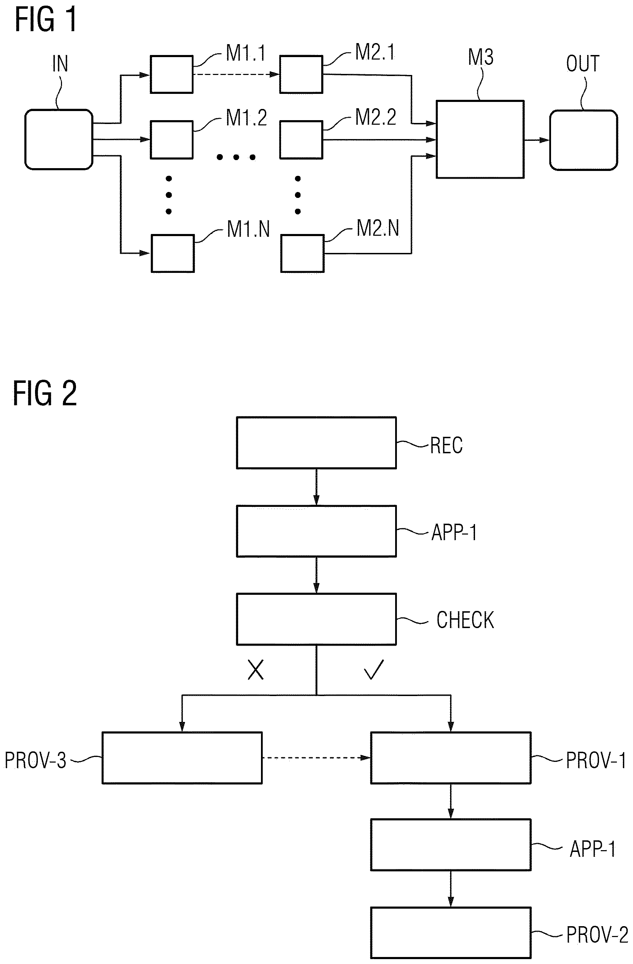

**Figure 1** displays a schematic flowchart of an exemplary stacked system as known in the prior art.

The stacked system comprises a plurality of functions M1.1, M1.2, M1.N, M2.1, M2.2, M2.N, M3. An input data IN is at least provided to a first layer of functions M1.1, M1.2, M1.N. The first layer of functions M1.1, M1.2, M1.N is applied to the input data, each of them thereby generating an intermediate output data. This intermediate output data can be used for further layers of functions M2.1, M2.2, M2.N as intermediate input data. In particular, a combination of two or more intermediate output data can be provided to one second layer function M2.1, M2.2, M2.N as intermediate input data. In particular, the intermediate input data of at least one of the second layer function M2.1, M2.2, M2.N can additionally comprise the input data IN.

A stacked system can comprise one or more layers of such functions M1.1, ..., M2.N.

The intermediate output data of the second layer functions M2.1, M2.2, M2.N is provided as intermediate input data to a further function M3 which is applied to the intermediate input data of the second layer functions M2.1, M2.2, M2.N. The intermediate input data of the further function M3 can additionally comprise the input data IN. The M3 generates the final output data OUT.

One or more of the functions M1.1, ..., M2.N, M3 can comprise a trained function. A general principle of a trained function is described above.

**Figure 2** displays a schematic flow chart of a first embodiment of the method for performing a check of a stacked system.

In a step of receiving REC input data IN, the input data IN is received from a medical system or a database. In particular, the input data IN can comprise a medical image data, in particular a medical radiology image data. The medical image data comprises an image of at least a part of a patient. The patient can be a human, an animal, or an object. The medical radiology image data can in particular comprise an x-ray image and/or a tomography image and/or a magnetic resonance image and/or a computed tomography image and/or an angiography image etc.

In a step of applying APP-1 a first function to the input data IN, the first function is applied to the input data IN, thereby generating an intermediate output data.

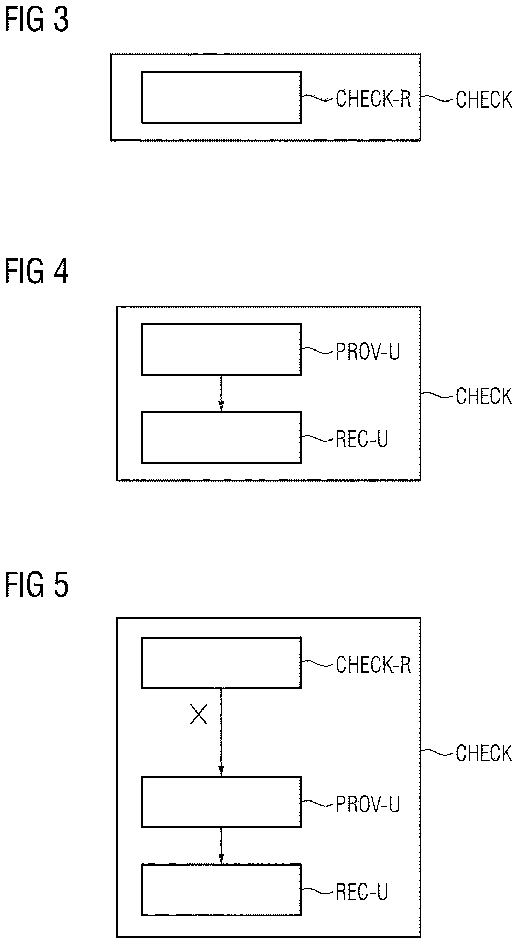

Based on this intermediate output data a check is performed in the step of performing CHECK a check. During this check it is checked whether the intermediate output data is correct or not. A more detailed description of different examples on how this check can be realized are described in figures 3 to 5.

When the intermediate output data seems to be correct, the check is positive otherwise it is negative.

If the check is positive, the intermediate output data is provided to a second function in the step of providing PROV-1 the intermediate output data at least as part of an intermediate input data. The intermediate input data can in particular also comprise the input data and intermediate output data of other functions which are comprised by the stacked system.

The second function is applied to the intermediate output data wherein an output data is generated.

In the step of providing PROV-2 the output data, the output data is provided for further use.

In particular, the output data can be the final output data OUT which is the final output of the stacked system. In this case the output data is not used anymore within the stacked system for generating further data. In this case the final output data can be provided in the step of providing PROV-1 the output data to a user and/or a database and/or an imaging device.

If the output data is provided to a user, it can in particular be displayed on a monitor.

If the output data is provided to a database, it can in particular be automatically integrated into a patient record related to the patient depicted on the corresponding input data. Alternatively or additionally, it can be used to automatically generate a diagnosis based on the provided output data. With other words the output data provided to the database can be used in further application to further analyze and/or use the output data in order to generate further knowledge and/or to store the output data in a structured, understandable manner and/or to generate parameter etc. which can be used for further data generation.

In particular, the output data can be provided to a medical imaging system which can use the output data to generate a new medical image data. In that case, the output data can comprise an imaging parameter. In x-ray imaging, the imaging parameter can e.g., be an acquisition time and/or a tube voltage and/or a tube current and/or a position of the patient etc.

Alternatively, the generated output data is not final. In particular the output data can be a second intermediate output data. This second intermediate output data can be provided to a third function at least as part of a second input data. In this case, the above-described steps of the check are repeated for the output generated by the third function.

The stacked system can comprise a plurality of such functions. Each of such functions can be receive an intermediate input data comprising e.g., the input data IN and/or an intermediate output data of one or more other functions. One of these functions is configured for generating the final output of the stacked system.

In particular, the first function can comprise a first trained function. In particular, the second function can comprise a second trained function. A general example for a trained function is provided above.

If the check is negative optionally a warning message is provided in the step of providing PROV-3 a warning message. The warning message can in particular be provided to a user. The warning message can be provided via a monitor and/via a speaker and/or via a flashlight etc. The warning message informs the user that the stacked system seems to not work properly based for the input data IN.

In particular, even if the check is negative, the intermediate output data can be provided to the second function as described above. The only difference can be, that the user is informed about potential problems which have been detected during the check.

**Figure 3** displays a schematic flow chart of a first embodiment of a step of performing CHECK a check.

In this embodiment, the check comprises a step of performing CHECK-R a rule-based check.

The rule-based check can be based on at least one hard coded rule. This rule depends on what is displayed by the input data. E.g., the rule can predefine the number of kidneys a patient typically has (two). And/or the rule can predefine the typical distances and the corresponding possible deviations between single vertebrae of a spine etc.

The rule-based check can in particular comprise a fourth trained function. This fourth trained function can be applied on the intermediate output data, wherein an information is generated which informs about how well the intermediate output data fits to the expectation. For that purpose, the fourth trained function can be trained based on a vast number of intermediate output data which is generated by applying the first function to a vast number of medical image data which depict the same part of a patient as the input data. The fourth trained function can then compare the intermediate output data with a vast number of intermediate output data and can therefore determine whether the intermediate output data is according to the expectation or whether there is some significant deviation from the expectation.

If there is a deviation of the rule and/or of the expectation, then the check is assumed to be negative. Otherwise, it is assumed to be positive.

**Figure 4** displays a schematic flow chart of a second embodiment of a step of performing CHECK a check.

The step of performing CHECK a check comprises a step of providing PROV-U the intermediate output data to a user and a step of receiving REC-U a user input based on the provided intermediate output data. The user input indicates whether the check is positive or negative. This can also be called user-based check.

In the step of providing PROV-U the intermediate output data to a user, the intermediate output data can in particular be provided via a user interface e.g., a monitor to the user. Like this the user can have a look at the intermediate output data and check whether it seems to be correct or not.

The user input can be provided by the user via the user interface. For that purpose, the user interface can comprise a computer mouse and/or a touch pad and/or a touch screen and/or a keyboard and/or a speaker for voice control etc.

Via the user input the user can indicate whether according to his perception, the intermediate output seems to be correct or not and like this he can indicate, whether the check is positive or negative.

**Figure 5** displays a schematic flow chart of a third embodiment of a step of performing CHECK a check.

According to this embodiment, the step of performing CHECK a check is a combination of the embodiments according to figures 3 and 4.

In a first step, a rule-based check is performed.

If this rule-based check is negative, the intermediate output data is provided to a user as described in figure 4 for a user-based check. Then the described user-based check is performed.

If the rule-based check is positive, then it is assumed that the complete check is positive, and the method continues as described in accordance with figure 1.

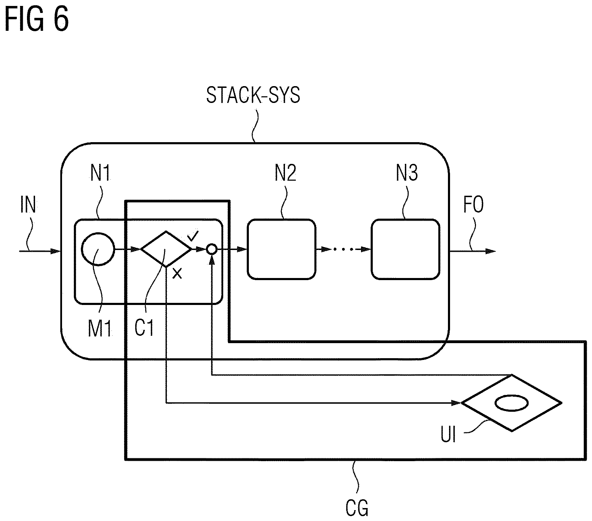

**Figure 6** displays a schematic flowchart of a stacked system STACK-SYS comprising a checking gate CG.

The stacked system comprises at least one first function M1 which in applied to an input data IN. The input data IN can be configured as described above. Being applied to the input data IN, the first function M1 generates an intermediate output data. The intermediate output data has to pass a checking gate CG. The checking gate CG can perform a rule-based check and/or a user-based check. The checks can be configured as described above. In the depicted embodiment a rule-based check C1 is combined with a user-based check UI. In a first step, the intermediate output data is evaluated whether it is in conformity with a predefined rule and/or with an expectation based on a variety of similar data. This is called rule-based check C1 and is described with more detail in accordance with figure 3. If the rule-based check C1 is positive, the intermediate output data is provided at least as part of an intermediate input data to a second function. If the rule-based check C1 is negative, the intermediate output data is further provided to a user via a user interface. The user can then perform a user-based check UI as described in accordance with figure 4. In this step the user can evaluate whether the negative result of the rule-based check C1 was correct or not. If the user also indicates that the check is negative, a warning message is provided, and the further data processing is stopped. If the user indicates that the check should be positive, then the intermediate output data is provided at least as part of an intermediate input data to the second function.

The first function M1 and the checking gate CG form a first complex N1. The second function is part of a second complex N2. The second complex can also comprise a further checking gate.

The stacked system STACK-SYS can comprise further complexes N3, comprising further functions. One of the functions provides the final output FO which is provided by the stacked system STACK-SYS as described above.

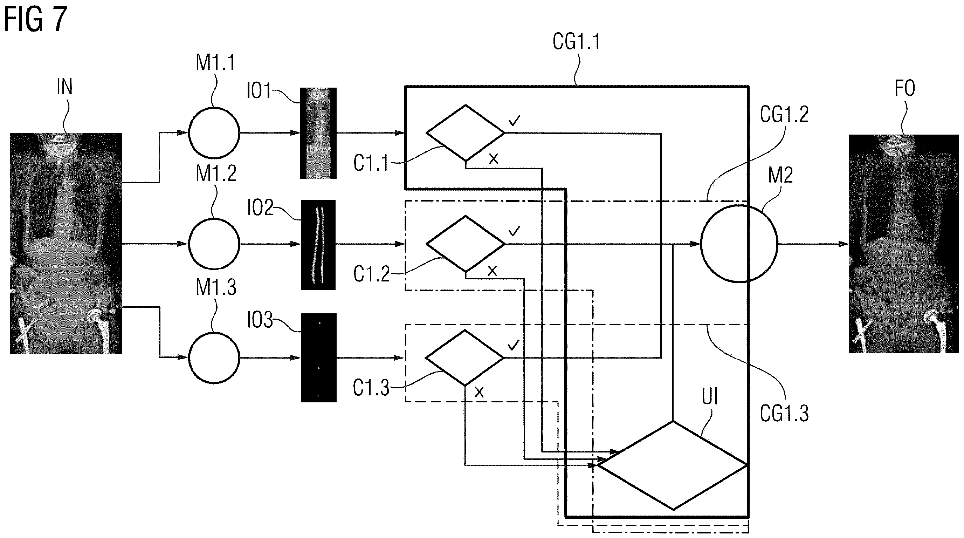

**Figure 7** displays a schematic flowchart of processing data by applying the method for performing a check of a stacked system STACKED-SYS.

The input data IN comprises an x-ray image of a human spine. The input data IN is provided to three first functions M1.1, M1.2, M1.3, Each of them generates a respective intermediate output IO1, IO2, IO3. The first function M1.1 provides a segmentation of the spine. The second first function M1.2 provides the outlines of the spine. The third first function M1.3 provides a position of specific vertebrae of the spine. The intermediate outputs IO1, IO2, IO3 of each first function M1.1, M1.2, M1.3 are provided to a corresponding checking gate CG1.1, CG1.2, CG1.3 each comprising a rule-based check C1.1, C1.2, C1.3 and a user-based check UI as described according to figure 6. If at least one of the user-based or the rule-based check is positive, the intermediate output data IO1, IO2, IO3 is provided at least as part of an intermediate input data to a second function M2. The second function M2 provides the final output FO. The final output FO comprises a form of the spine in the x-ray image.

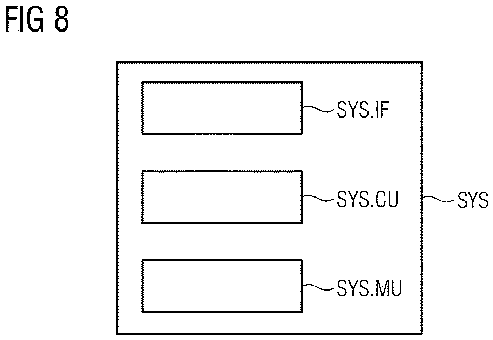

**Figure 8** displays a system SYS for performing a check of a stacked system STACK-SYS.

The displayed system SYS is configured to execute a method according to the invention for performing a check of a stacked system STACK-SYS. The system SYS comprises an interface SYS.IF, a computation unit SYS.CU, and a memory unit SYS.MU.

The system SYS can in particular be a computer, a microcontroller, or an integrated circuit. Alternatively, the system SYS can be a real or a virtual network of computers (a technical term for a real network is "cluster", a technical term for a virtual network is "cloud"). The system SYS can also be designed as virtual system that is executed on a computer, a real network of computers or a virtual network of computers (a technical term is "virtualization").

An interface SYS.IF can be a hardware or software interface (for example PCI bus, USB or Firewire). A computation unit SYS.CU can have hardware elements or software elements, for example a microprocessor or a so-called FPGA (acronym for "field programmable gate way"). A memory unit SYS.MU can be implemented as a non-permanent working memory (random access memory, RAM for short) or as a permanent mass storage device (hard disk, USB stick, SD card, solid state disk).

The interface SYS.IF can in particular comprise a plurality of sub-interfaces which carry out different steps of the respective method. In other words, the interface SYS.IF can also be understood as a plurality of interfaces SYS.IF. The computation unit SYS.CU can in particular comprise a plurality of sub-computing units which carry out different steps of the respective method. In other words, the computation unit SYS.CU can also be understood as a plurality of computation units SYS.CU.

Wherever not already described explicitly, individual embodiments, or their individual aspects and features, can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

## Claims

1. Computer implemented method for performing a check of a stacked system (STACK-SYS) comprising the following steps:
- receiving (REC)input data (IO),
- applying (APP-1) a first function (M1, M1.1, M1.2, M1.3) to the input data,
wherein an intermediate output data (IO1, IO2, IO3) is generated,
- performing (CHECK) a check based on the intermediate output data (IO1, IO2, IO3),
if the check is positive,
- providing (PROV-1) the intermediate output data (IO1, IO2, IO3) to a second function (M2) at least as part of an intermediate input data,
- applying (APP-2) the second function (M2) to the intermediate input data, wherein an output data is generated,
- providing (PROV-2) the output data.

2. Method according to claim 1,
wherein if the check is negative, the method comprises the following step:
- providing (PROV-3) a warning message.

3. Method according to one of the preceding claims,
wherein the first function (M1, M1.1, M1.2, M1.3) comprises a first trained function and/or
wherein the second function (M2) comprises a second trained function.

4. Method according to one of the preceding claims,
wherein providing the output data comprises:
- providing the output data as final output data (FO) to a user and/or a database and/or an imaging device, or
- providing the output data as second intermediate input data to a third function after performing a check.

5. Method according to claim 4,
further comprising at least one of the following steps if the output data is provided as final output data:
- acquiring medical image data with a medical imaging system based on the final output data (FO),
- automatically including the final output data (FO) in a patient record,
- providing a diagnosis based on the final output data (FO).

6. Method according to one of the preceding claims,
wherein the check is rule-based.

7. Method according to one of the preceding claims,
wherein performing the check comprises the following steps:
- providing (PROV-U) the intermediate output (IO1, IO2, IO3) data to a user,
- receiving (REC-U) a user input based on the provided intermediate output data (IO1, IO2, IO3),
wherein the user input indicates whether the check is positive or negative.

8. Method according to claim 7 in combination with claim 6, wherein the intermediate output data (IO1, IO2, IO3) is provided to the user if the rule-based check is negative.

9. Method according to one of the claims 7 or 8,
wherein the user input comprises one of the following:
- an agreement of the user with the intermediate output data (IO1, IO2, IO3), indicating a positive check,
- a disagreement of the user with the intermediate output data (IO1, IO2, IO3), indicating a negative check,
- a correction of the intermediate output data (IO1, IO2, IO3), wherein the corrected intermediate output data replaces the intermediate output data (IO1, IO2, IO3) during the following procedure, indicating a positive check based on the corrected intermediate output data.

10. Method according to one of the preceding claims,
wherein the input data (IO) comprises a medical image data, in particular a medical radiology image data.

11. Method according to claim 10,
wherein the input data (IO) comprises a medical image data of a spine,
wherein the intermediate output data (IO1, IO2, IO3) comprises at least one of the following:
- a position of at least one vertebrae of the spine,
- a segmentation of the spine,
- a baseline describing the form of the spine.

12. A stacked system (STACK-SYS) comprising
- at least one first function (M1, M1.1, M1.2, M1.3),
- at least one checking gate (CG), and
- a second function (M2),
wherein the at least one first function (M1) is configured for being applied to input data (IN), wherein intermediate output data (IO1, IO2, IO3) is generated,
wherein the checking gate (CG) is configured for performing a check of the intermediate output data (IO1, IO2, IO3),
wherein the second function (M2) is configured for being applied to the intermediate output data if the check is positive,
wherein the second function (M2) is configured for being applied to the intermediate output data (IO1, IO2, IO3) thereby generating an output data.

13. A stacked system (STACK-SYS) according to claim 12, wherein the check is rule-based and/or based on a user input.

14. A system (SYS) for performing a check of a stacked system (STACK-SYS) comprising an interface (SYS.IF) and a computation unit (SYS.CU), wherein the interface (SYS.IF) and the computation unit (SYS.CU) are configured to perform the following steps:
- receiving input data (IO),
- applying a first function (M1, M1.1, M1.2, M1.3) to the input data (IO),
wherein an intermediate output data (IO1, IO2, IO3) is generated,
- performing a check based on the intermediate output data (IO1, 102, 103),
if the check is positive,
- providing the intermediate output data (IO1, IO2, IO3) to a second function (M2) at least as part of an intermediate input data,
- applying the second function (M2) to the intermediate input data, wherein an output data is generated,
- providing the output data, or
if the check is negative,
- providing a warning message.

15. A computer program product comprising program elements, directly loadable into a memory unit of a system (SYS), which induces the system (SYS) to execute the method according to any of claims 1 to 11 when the program elements are executed by the system (SYS).

16. A computer-readable storage medium comprising program elements which are readable and executable by a system (SYS), to execute the method of one of the claims 1 to 11, when the program elements are executed by the system (SYS).
